(19) Europäisches Patentamt / European Patent Office / Office européen des brevets

(11) **EP 3 783 343 B1**

(12) **EUROPÄISCHE PATENTSCHRIFT**

(45) Veröffentlichungstag und Bekanntmachung des Hinweises auf die Patenterteilung:
**25.08.2021 Patentblatt 2021/34**

(51) Int Cl.:
*G01N 22/00* (2006.01)        *G01N 33/18* (2006.01)
*G01F 1/74* (2006.01)

(21) Anmeldenummer: **20169149.0**

(22) Anmeldetag: **09.04.2020**

(54) **BESTIMMUNG EINES MISCHUNGSVERHÄLTNISSES**

DETERMINATION OF A MIXING RATIO

DÉTERMINATION D'UN RAPPORT DE MÉLANGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.08.2019 EP 19193137**

(43) Veröffentlichungstag der Anmeldung:
**24.02.2021 Patentblatt 2021/08**

(73) Patentinhaber: **Siemens Schweiz AG**
**8047 Zürich (CH)**

(72) Erfinder:
• **Konrad, Hilmar**
**6340 Baar (CH)**
• **Petry, Karl-Heinz**
**8864 Reichenburg (CH)**
• **Weiers, Tilman**
**6300 Zug (CH)**

(74) Vertreter: **Maier, Daniel Oliver**
**Siemens AG**
**Postfach 22 16 34**
**80506 München (DE)**

(56) Entgegenhaltungen:
EP-A2- 2 520 148        WO-A1-2018/078401
US-A1- 2009 126 502

## Beschreibung

Hintergrund

Die vorliegende Offenbarung behandelt die Bestimmung des Mischungsverhältnisses einer Rohrleitungen durchströmenden Flüssigkeit in Anlagen der Heizungs-, Lüftungs-, Klima- und Kältetechnik mittels RADAR. Die vorliegende Offenbarung betrifft weiterhin eine Messeinrichtung sowie ein Verfahren zur Bestimmung des Mischungsverhältnisses einer Flüssigkeit.

In dieser Offenbarung geht es insbesondere um die Bestimmung von Mischungsverhältnissen in intelligenten (smarten) Durchflussventilen. Dabei ist oft ein Mischungsverhältnis einer Mischung aus Glycol und Wasser zu bestimmen. Die Kenntnis des Glycol-Gehaltes in einem Gemisch aus Wasser und Glycol ermöglicht eine adäquate Verarbeitung der Wärmeübertragung durch das Ventil.

Eine internationale Patentanmeldung WO 2012/065276 A1 wurde am 19. Oktober 2011 angemeldet. Die Anmeldung wurde veröffentlicht am 24. Mai 2012. WO 2012/065276 A1 behandelt die Bestimmung eines Wärmestromes aus einer wärmetransportierenden Flüssigkeit. Gemäss WO 2012/065276 A1 sind in einer Vorrichtung 10 zur Messung eines Wärmeflusses zwei Ultraschall-Wandler 14, 15 angeordnet. Die Ultraschall-Wandler kommunizieren mit einem Regler 19. Der Regler 19 wiederum ist mit einer Auswerteeinheit 20 verbunden. Zusätzlich umfasst die Vorrichtung 10 einen Temperaturfühler 17, welcher zwischen den beiden Ultraschall-Wandlern angeordnet ist.

In der Vorrichtung 10 aus WO 2012/065276 A1 wird nun anhand des Temperaturfühlers 10 die absolute Temperatur eines Fluides bestimmt. Zugleich wird anhand der Ultraschall-Wandler 14, 15 die Schallgeschwindigkeit im Fluid gemessen. Aus der absoluten Temperatur und aus der gemessenen Schallgeschwindigkeit kann nun auf Dichte und Mischungsverhältnis eines Wasser-Glycol-Gemisches geschlossen werden.

Am 29. März 2007 wurde in Deutschland eine Patentanmeldung DE 10 2007 015 609 A1, Kälte- oder Wärmezählereinrichtung zur Ermittlung des Energieverbrauchs in einem Temperierungskreislauf, eingereicht. Die gleiche Patentanmeldung wurde am 9. Oktober 2008 veröffentlicht. DE 10 2007 015 609 A1 offenbart eine Messvorrichtung 2 mit Ultraschall-Messköpfen 4 zum Bestimmen von Durchflussmengen. Die Messvorrichtung 2 umfasst auch zwei Temperatursonden 9 zum Erfassen des Temperaturabfalls zwischen dem Vorlauf- und dem Rücklaufende. Die Temperatursonden 9 sowie die Ultraschall-Messköpfe 4 sind mit einer Steuerung 12 verbunden.

Die Messvorrichtung 2 von DE 10 2007 015 609 A1 bietet ein Mikroanemometer 13. Das Mikroanemometer 13 ist zwischen Vorlauf- und Rücklaufseite angeordnet und verbindet sich ebenfalls mit der Steuerung 12. Eine Schätzung k in Bezug auf die spezifische Wärme ergibt sich aus den vom Mikroanemometer 13 erfassten Werten. Das Mikroanemometer 13 ermöglicht es somit, Werte von k in eine Wärmestromschätzung einzubeziehen. Es ist vorstellbar, aus den Werten von k auf die Zusammensetzung eines Wasser-Glycol-Gemisches zu schliessen.

Abgesehen von den Ansätzen aus DE 10 2007 015 609 A1 und aus WO 2012/065276 A1 ist eine manuelle Eingabe möglich. Anstatt ein Mischungsverhältnis automatisch zu bestimmen, erfordert der manuelle Ansatz eine Eingabe durch einen Benutzer. Der Ansatz setzt eine hinreichende Kenntnis des Mischungsverhältnisses eines Wasser-Glycol-Gemisches in den Rohrleitungen einer Heizungs-, Lüftungs- und Klimaanlage voraus. Der manuelle Ansatz ist anfällig für fehlerhafte Eingaben durch einen Benutzer.

Eine Patentanmeldung WO2018/078401A1 wurde eingereicht am 31. Oktober 2017 durch UNIV HERIOT WATT. Die Anmeldung wurde veröffentlicht am 3. Mai 2018. WO2018/078401A1 nimmt eine Priorität vom 31. Oktober 2016 in Anspruch. WO2018/078401A1 befasst sich mit einem System zur Detektion von Materialeigenschaften.

Eine Patentanmeldung US2009/126502A1 wurde eingereicht am 18. Dezember 2006 durch SKJAELDAL INGVE MORTEN und WEE ARNSTEIN. Die Anmeldung wurde veröffentlicht am 21. Mai 2009. US2009/126502A1 nimmt eine Priorität vom 5. Mai 2006 in Anspruch. US2009/126502A1 offenbart tomographische, mehrphasige Flussmessungen.

Eine Patentanmeldung EP2520148A2 wurde eingereicht am 27. April 2012 durch MSO MESSTECHNIK UND ORTUNG GMBH. Die Anmeldung wurde veröffentlicht am 7. November 2012. EP2520148A2 behandelt ein Verfahren zur Messung eines Gutstroms mittels Microwellen, sowie eine Sensoranordnung und eine Vorrichtung mit einer Sensoranordnung. EP2520148A2 nimmt eine Priorität vom 2. Mai 2011 in Anspruch.

Die vorliegende Offenbarung lehrt eine Klassifizierung von Flüssigkeiten, insbesondere von Wasser-Glycol-Gemischen, welche ohne aufwändige Ultraschall-Sensoren auskommt. Eine Klassifizierung entsprechend der vorliegenden Offenbarung vermeidet Fehler durch unkorrekte Eingaben von Benutzern.

Kurzfassung

Aus einem Projekt Soli (https://atap.google.com/soli/, abgerufen am 6. August 2019) ist ein Miniatur-Radarsensorsystem bekannt. Jenes Miniatur-Radarsensorsystem ist ursprünglich zur Gestenerkennung entwickelt worden. Gemäss der vorliegenden Offenbarung wird anstelle der radargestützten Bewegungserfassung von Fingern zur Gestenerkennung ein Mischungsverhältnis bestimmt. Der Sensor hat Seitenabmessungen von zehn Millimetern versus acht Millimetern (10 mm x 8 mm). Es kommen Millimeter-Radarwellen bei sechzig Gigahertz (60 GHz) zum Einsatz. Die Leistungsaufnahme beträgt dreihundert Milliwatt (300 mW). Die Reichweite des Sensors liegt bei zehn Metern (10 m). Weitere technische Details zum Soli-Sensor sind unter anderem ersichtlich aus in einem Artikel von Jaime Lien, Nicholas Gillian, M. Emre Karagozler, Patrick Amihood, Carsten Schwesig, Erik Olson, Hakim Raja und Ivan Poupyrev. Jener Artikel ist im Juli 2016 veröffentlicht worden in ACM Transactions on Graphics, Band 35, Nummer 4, Artikel 142. Der Artikel trägt den Titel Soli: Ubiquitous Gesture Sensing with Millimeter Wave Radar.

Es ist zudem ein Ziel der vorliegenden Offenbarung, eine robuste Anordnung zur Klassifizierung eines Wasser-Glycol-Gemisches bereitzustellen. Dazu wird ein Radarsensorsystem benachbart zu einem Leitungsrohr angeordnet. Das Radarsensorsystem ist mithin von dem zu untersuchenden Fluid räumlich getrennt.

Weiterhin ist ein Ziel der vorliegenden Offenbarung, die Untersuchung eines Wasser-Glycol-Gemisches anhand kommerziell erhältlicher Komponenten zu vollziehen. Es wird aus diesem Grund auf einen kommerziell erhältlichen Radarsensor zurückgegriffen. Eine Klassifizierung gemäss der vorliegenden Offenbarung eignet sich für den industriellen Einsatz beispielsweise in Ventilen der Heizungs-, Lüftungs- und Klimatechnik.

Es ist ferner ein Ziel der vorliegenden Offenbarung, eine Bestimmung eines Mischungsverhältnisses bereitzustellen, welche für verschiedenste Fluide angewendet werden kann. So ist die offenbarte Klassifizierung nicht beschränkt auf Mischungen aus Wasser und Glycol. Stattdessen eignet sich die Klassifizierung auch zur Erkennung gefährlicher Flüssigkeiten und/oder gefährlicher Bestandteile in einem Gemisch.

Es ist auch ein Ziel der vorliegenden Offenbarung, eine Einrichtung bereitzustellen, wobei die Einrichtung eine digitale Recheneinheit zum genauen Berechnen eines Mischungsverhältnisses eines Gemisches mindestens zweier Fluide nutzt. Es ist darüber hinaus ein Ziel der vorliegenden Offenbarung, eine Einrichtung bereitzustellen, wobei die Einrichtung die arithmetischen Funktionen einer digitalen Recheneinheit weitgehend nutzt zur präzisen Berechnung eines Mischungsverhältnisses eines Gemisches mindestens zweier Fluide.

Es ist weiterhin ein Ziel der vorliegenden Offenbarung, Mischungsverhältnisse möglichst genau zu bestimmen. Dazu wird eine Anordnung bereitgestellt, welche Störungen in einem Leitungsabschnitt durch Reflexionen unterdrückt.

Es ist darüber hinaus ein Ziel der vorliegenden Offenbarung, Ausfälle an Geräten wie beispielsweise an Ventilen der Heizungs-, Lüftungs- und Klimatechnik zu erkennen. Beispielsweise können anhand des Radarsensors gewonnene Messwerte auf Plausibilität geprüft werden. Gegebenenfalls wird ein Signal an einen Benutzer abgesetzt, wonach eine Einrichtung zu warten oder zu reparieren ist. Ebenso ist es möglich, im Fall unplausibler Messwerte ein Ventil zu schliessen. Damit wird ein Heizungs-, Lüftungs- und Klimasystem verriegelt.

Kurze Beschreibung der Figuren

Verschiedene Details werden dem Fachmann anhand der folgenden detaillierten Beschreibung zugänglich. Die einzelnen Ausführungsformen sind dabei nicht einschränkend. Die Zeichnungen, welche der Beschreibung beigefügt sind, lassen sich wie folgt beschreiben:

FIG 1 veranschaulicht einen Leitungsabschnitt mit einem Radarsensorsystem.

FIG 2 zeigt wie FIG 1 einen Leitungsabschnitt mit einem Radarsensorsystem, wobei gegenüber dem Radarsensorsystem eine Schicht aus radarwellenabsorbierendem Material angebracht ist.

FIG 3 veranschaulicht schematisch die Steuer- und/oder Regeleinheiten für das Radarsensorsystem.

FIG 4 zeigt weitere Details des Radarsensorchips.

FIG 5 veranschaulicht anhand eines Diagramms einen Zusammenhang zwischen Reflexionsfaktor und Frequenz.

Detaillierte Beschreibung

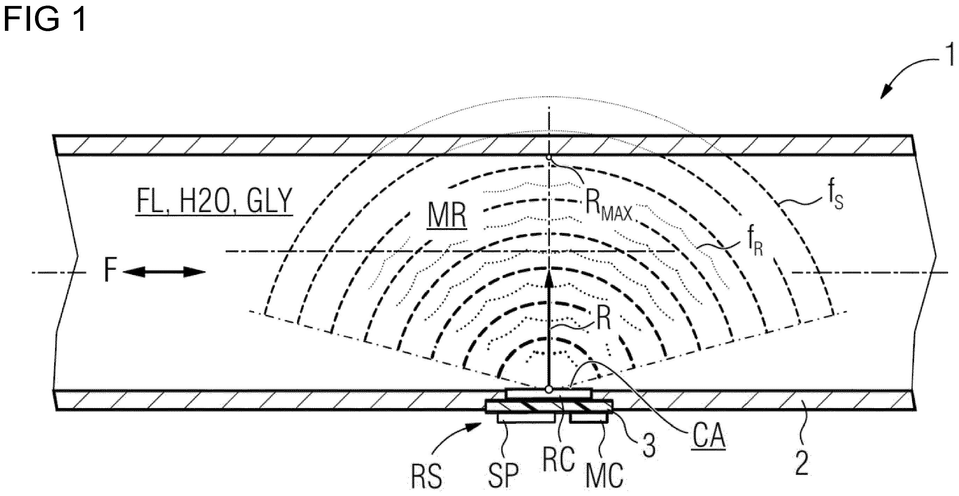

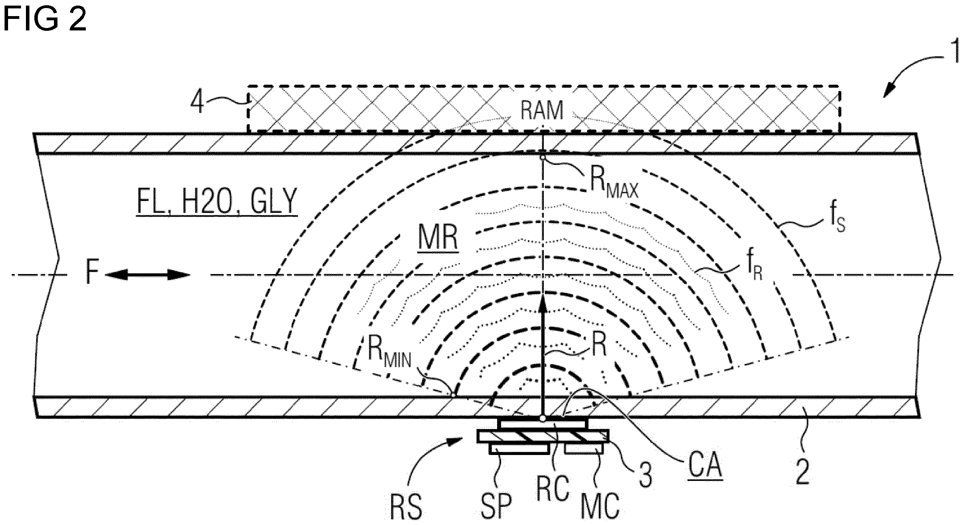

FIG 1 veranschaulicht das grundlegende Messprinzip. Millimeter-Radarwellen $f_S$ mit einer Frequenz von beispielhaft sechzig Gigahertz und mit einer entsprechenden Wellenlänge von fünf Millimetern und weniger werden in den Innenraum MR eines Leitungsabschnittes 2 eingestrahlt. Dieser Innenraum MR kann auch als Messbereich bzw. Messraum bezeichnet werden. Mit dem Bezugszeichen R ist ein radialer Abstand von einer Sensoraussenseite CA des Radarsensorchips RC, insbesondere von der Flächenmitte der Sensoraussenseite CA, bezeichnet. Im Vorgriff auf die nachfolgende FIG 2 ist mit $R_{MIN}$ ein minimaler radialer Abstand bezeichnet, ab welchem die vom Radarsensorchip RC ausgesendeten Millimeter-Radarwellen $f_S$ ausschliesslich durch das zu untersuchende Gemisch FL hindurchverlaufen. Mit $R_{MAX}$ ist in entsprechender Weise ein maximaler radialer Abstand bezeichnet, bis zu welchem die ausgesendeten Millimeter-Radarwellen $f_S$ ausschliesslich noch durch das zu untersuchende Gemisch FL hindurchverlaufen.

Dabei kommt ein miniaturisierter Radarsensorchip RC zum Einsatz. Der Radarsensorchip RC ist benachbart zum Leitungsabschnitt 2 platziert. Das Leitungsabschnitt 2 selbst ist vorzugsweise aus einem Material hergestellt, welches für die oben genannten Millimeter-Radarwellen im Wesentlichen transparent ist. Das Material kann beispielsweise ein Kunststoff oder eine Keramik sein. Ein Gemisch FL, wie zum Beispiel eine Mischung aus Wasser und Glycol, fliesst durch den Leitungsabschnitt 2. Das Gemisch FL streut dabei die in den Innenraum MR des Leitungsrohres 2 bzw. Leitungsabschnittes eingestrahlten Millimeter-Radarwellen $f_S$. Der Radarsensorchip RC empfängt die gestreuten Millimeter-Radarwellen $f_R$ und verarbeitet diese signaltechnisch.

Die Streueigenschaften hängen von den elektromagnetischen Eigenschaften des Fluides FL ab. Entsprechend kann das Gemisch FL basierend auf dessen Streueigenschaften klassifiziert werden.

Als Gemisch FL sind beispielsweise Wasser und/oder eine Wassermischung vorgesehen. Insbesondere sind Mischungen aus Wasser und mindestens einer weiteren Substanz ausgewählt aus:

- Calciumchlorid,
- Ethanol,
- Ethylenglycol,
- Glycerin,
- Kaliumacetat,
- Kaliumformiat,
- Magnesiumchlorid,
- Methanol,
- Natriumchlorid und/oder
- 1,2-Propandiol

vorgesehen.

Weiterhin kann das Fluid ein Kühlmittel ausgewählt aus:

- R-401A,
- R-404A,
- R-406A,
- R-407A,
- R-407C,
- R-408A,
- R-409A,
- R-410A,
- R-438A,
- R-500, und/oder
- R-502

umfassen. Die vorstehenden Listen sind nicht abschliessend.

Es wird ein sogenannter komplexer Reflexionsfaktor $\rho_f$ analysiert. Insbesondere werden die Veränderungen des komplexen Reflexionsfaktors $\rho_f$ mit der Materialzusammensetzung analysiert. Es ist vorgesehen, die Streueigenschaften eines Fluides FL im relevanten Frequenzbereich zu analysieren.

Ferner können Dämpfungen von Hochfrequenzsignalen Hinweise auf Flüssigkeitsarten geben. Beispielsweise kann eine Flüssigkeit wie Milch auf diese Weise von Leitungswasser unterschieden werden.

Ausserdem können Veränderungen der dielektrischen Eigenschaften von Lösungen mit unterschiedlichen Glucose-Werten erkannt werden. Auf diese Weise ist es möglich, zwischen verschiedenen Konzentrationen zu unterscheiden. So sind Millimeterwellen zur Glucose-Erkennung in biologischen Medien bei Konzentrationen ähnlich den Blutzuckerkonzentrationen diabetischer Patienten geeignet.

Erfindungsgemäß wird vorgeschlagen, frequenzmodulierte Millimeter-Radarwellen $f_S$ mit einem vorgegebenen Frequenzhub, also in einem vorgegebenen Frequenzbereich $\Delta f$, im Sinne eines Chirp-Signales in den Messbereich MR einzustrahlen. Derartige (kontinuierlich) frequenzmodulierte Millimeter-Radarwellen $f_S$ können z.B. sogenannte FMCW-Millimeter-Radarwellen $f_S$ sein. Es werden dann die an dem Gemisch FL sowie an dem Material des Leitungsabschnittes 2 zurückgestreuten, entsprechend frequenzmodulierten Millimeter-Radarwellen $f_R$ anhand eines Empfangsantennensignales Rx0' mit dem Sendeantennensignal Tx0' zu einem Zwischenfrequenzsignal (herunter)gemischt. Anschliessend wird das Zwischenfrequenzsignal in ein zugehöriges Frequenzspektrum umgewandelt, wie z.B. mittels einer Fouriertransformation. Der frequenzabhängige Reflexionsfaktor $\rho_f$ ist dann aus dem Frequenzspektrum des heruntergemischten Zwischenfrequenzsignales ermittelbar.

Durch das Heruntermischen des Empfangsantennensignales Rx0' ist vorteilhaft eine vereinfachte elektronische Weiterverarbeitung in einem deutlich niedrigeren Frequenzband möglich.

Um mögliche messtechnische nachteilige Auswirkungen von Reflexionen der ausgesendeten Millimeter-Radarwellen $f_S$ an dem Werkstoff des Leitungsabschnittes 2 zu minimieren, kann z.B. ein Beginn des Zwischenfrequenzsignales «weggeschnitten» werden. Das weggeschnittene Signal entspricht in zeitlicher Hinsicht den von der Wandung des Leitungsabschnittes 2 direkt am Radarsensorchip RC reflektierten Radarwellen $f_R$ (siehe FIG 2). Also kann der zeitliche Abschnitt des Zwischenfrequenzsignales, welcher reflektierten Radarwellen $f_R$ innerhalb des Mindestabstandes $R_{MIN}$ von der Sensorchipaussenseite zugeordnet werden kann, unberücksichtigt bleiben. In entsprechender Weise kann das Ende des Zwischenfrequenzsignales «abgeschnitten» werden, der in zeitlicher Hinsicht den von der gegenüberliegenden Wandung des Leitungsabschnittes 2 reflektierten Radarwellen $f_R$ entspricht (siehe FIG 2). Es kann der zeitliche Abschnitt des Zwischenfrequenzsignales, der reflektierten Radarwellen $f_R$ grösser als der Maximalabstand $R_{MAX}$ von der Sensorchipaussenseite zugeordnet werden kann, unberücksichtigt bleiben.

Alternativ kann das komplette Zwischenfrequenzsignal in das zugehörige Frequenzspektrum umgewandelt werden. Es können dann die Frequenzbereiche im Frequenzspektrum unberücksichtigt bleiben, die direkt proportional zum Minimalabstand $R_{MIN}$ und Maximalabstand $R_{MAX}$ sind. Im Beispiel der FIG 2 werden an dem Gemisch FL reflektierte Radarwellen $f_R$ nur für radiale Abstandswerte R - gemessen von der Sensorchipaussenseite CA - berücksichtigt, die grösser sind als der Mindestabstand $R_{MIN}$ und kleiner sind als der Maximalabstand $R_{MAX}$.

Optional kann eine radarwellenabsorbierende Schicht 4 angeordnet werden. Eine solche radarwellenabsorbierende Schicht 4 zeigt FIG 2. Die Schicht 4 unterdrückt Störungen. Sie kann derart angeordnet sein, dass sie mindestens Teile der Leitung 2 von aussen umschliesst. Die radarwellenabsorbierende Schicht 4 kann auch innerhalb der Leitung angeordnet sein. Es ist weiterhin vorgesehen, dass die Wand bzw. die Wandung der Leitung ein radarwellenabsorbierendes Material umfasst.

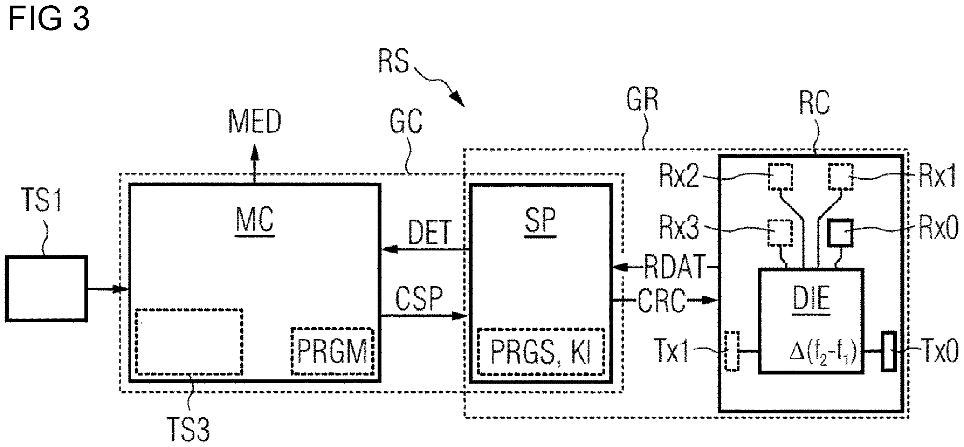

FIG 3 zeigt ein Radarsensorsystem RS umfassend einen Radarsensorchip mit integriertem Signalprozessor GR. Radarsensorsystem RS umfasst weiterhin Mikrocontroller mit integriertem Signalprozessor GC. Der Mikrocontroller mit integriertem Signalprozessor GC erfasst anhand eines ersten Temperatursensors TS1 die Temperatur eines Gemisches FL im Leitungsabschnitt 2.

Anhand einer Schnittstelle gibt der Mikrocontroller mit integriertem Signalprozessor GC digitale oder analoge Informationen zum Typ des Gemisches FL aus. Insbesondere gibt der Mikrocontroller mit integriertem Signalprozessor GC digitale oder analoge Informationen zum Mischungsverhältnis des Gemisches FL aus.

Ein vom Mikrocontroller mit integriertem Signalprozessor GC umfasster Mikrocontroller MC sendet dazu Steuerdaten CSP an einen Signalprozessor SP. Im Gegenzug sendet der Signalprozessor SP ein Detektionsergebnis DET an den Mikrocontroller MC.

Vorteilhaft umfasst der Mikrocontroller mit integriertem Signalprozessor GC auch den Signalprozessor SP. In einer kompakten Ausführungsform sind der Mikrocontroller MC und der Signalprozessor SP auf demselben Chip angeordnet. Der Mikrocontroller MC und der Signalprozessor SP sind in diesem Fall Teile eines Ein-Chip-Systems.

In einer Ausführungsform umfasst der Mikrocontroller MC einen Speicher. Im Speicher des Mikrokontrollers MC können beispielsweise Tabellenwerte zur Bestimmung des Mischungsverhältnisses eines Gemisches FL abgelegt sein. Der Speicher des Mikrocontrollers MC ist vorteilhaft nichtflüchtig.

Es ist weiterhin vorgesehen, dass der Mikrocontroller MC über eine arithmetisch logische Einheit verfügt. Die arithmetisch logische Einheit des Mikrocontrollers MC führt Berechnungen durch, wie sie beispielsweise zur Ermittlung des Mischungsverhältnisses eines Gemisches FL notwendig sind.

Der Signalprozessor SP empfängt seinerseits Daten RDAT vom Radarsensorchip RC. Zugleich steuert der Signalprozessor SP anhand von Steuersignalen CRC den Radarsensorchip RC. So ist vorgesehen, dass der Signalprozessor RC Steuersignale CRC wie Betriebsarten, Frequenzen und/oder Frequenzhub an den Radarsensorchip RC sendet.

Vorteilhaft umfasst der Radarsensorchip mit integriertem Signalprozessor GR auch den Signalprozessor SP. In einer weiteren kompakten Ausführungsform sind der Radarsensorchip RC und der Signalprozessor SP auf demselben Chip angeordnet. Der Radarsensorchip RC und der Signalprozessor SP sind in diesem Fall Teile eines Ein-Chip-Systems.

Es ist ferner vorgesehen, dass der Mikrocontroller MC und der Signalprozessor SP und der Radarsensorchip RC auf demselben Chip angeordnet sein können. Der der Mikrocontroller MC und der Signalprozessor SP und der Radarsensorchip RC sind in diesem Fall Teile eines Ein-Chip-Systems.

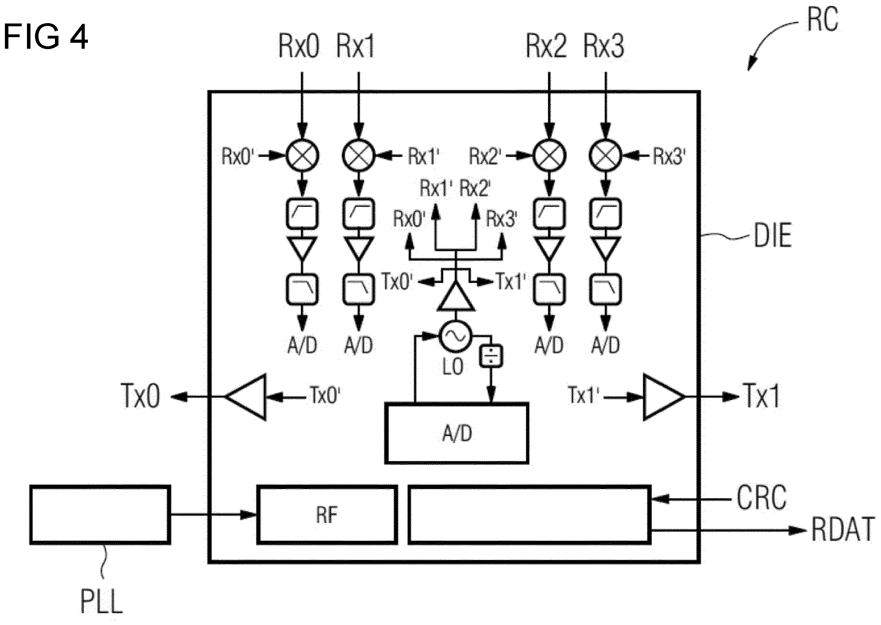

Details des Radarsensorchips RC veranschaulicht FIG 4. Der Radarsensorchip RC verfügt über mindestens eine Empfangsantenne Rx0 - Rx3. Die mindestens eine Empfangsantenne Rx0 - RX3 ist angeordnet zum Empfang radiofrequenter Signale aus dem Leitungsabschnitt 2. Die mindestens eine Empfangsantenne Rx0 - RX3 ist insbesondere angeordnet zum Empfang von Millimeter-Radarwellen aus dem Leitungsabschnitt 2.

Es ist ferner vorgesehen, dass der Radarsensorchip RC mindestens zwei Empfangsantennen Rx0 - RX3 umfasst. Vorzugsweise umfasst der Radarsensorchip RC sogar drei oder vier Empfangsantennen Rx0 - RX3.

Der Radarsensorchip RC verfügt ausserdem über mindestens eine Sendeantenne Tx0, Tx1. Die mindestens eine Sendeantenne Tx0, Tx1 ist angeordnet zum Einstrahlen radiofrequenter Signale in den Leitungsabschnitt 2. Die mindestens eine Sendeantenne Tx0, Tx1 ist insbesondere angeordnet zum Einstrahlen von Millimeter-Radarwellen in den Leitungsabschnitt 2.

Vorzugsweise umfasst der Radarsensorchip RC eine Hochfrequenzstufe RF. Die Hochfrequenzstufe RF kommuniziert ihrerseits mit einer Phasenregelschleife PLL. Jene Phasenregelschleife PLL kann zudem einen Zeitgeber umfassen. In einer integrierten Ausführungsform sind der Radarsensorchip RC und die Phasenregelschleife PLL auf demselben Chip angeordnet. Der Radarsensorchip RC und die Phasenregelschleife PLL sind in diesem Fall Teile eines Ein-Chip-Systems.

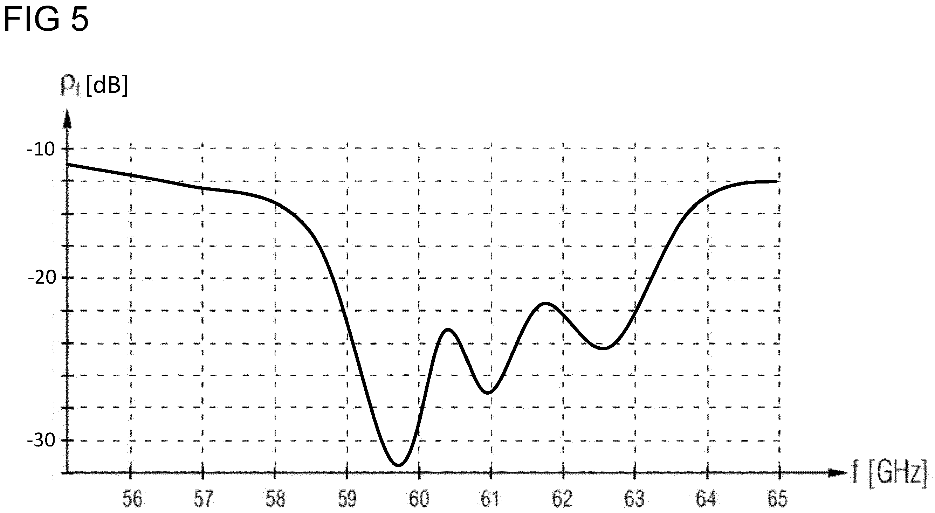

Einen beispielhaften Verlauf des Reflexionsfaktors $\rho_f$ über der Frequenz zeigt FIG 5. Der Reflexionsfaktor $\rho_f$ wird zur Bestimmung des Mischungsverhältnisses des Gemisches FL verwendet. Der Reflexionsfaktor ist definiert als Verhältnis von reflektiertem $V_r$ zu eingestrahltem Signal $V_h$:

$$\rho_f = V_r/V_h.$$

Das reflektierte Signal $V_r$ und das eingestrahlte Signal $V_h$ sind im Allgemeinen komplexe Grössen. Deswegen wird der Betrag des Reflexionsfaktors $|\rho_f|$ häufig als Funktion des Stehwellenverhältnisses SWR angegeben:

$$|\rho_f| = (SWR - 1)/(SWR + 1).$$

Vorteilhaft wertet das Radarsensorsystem RS den Betrag und/oder den Realteil des Reflexionsfaktors $\rho_f$ aus. Beispielsweise kann anhand des Reflexionsfaktors $\rho_f$ und anhand einer in einem Speicher des Radarsensorsystems RS hinterlegten Zuordnungstabelle ein Mischungsverhältnis zugeordnet werden. Gegebenenfalls kommt zusätzlich zur hinterlegten Tabelle eine Interpolation, insbesondere eine lineare Interpolation, zwischen Tabellenwerten zum Einsatz.

Im hier verwendeten Sinn bedeuten die Begriffe "etwa" und "im Wesentlichen", wenn diese in Verbindung mit einem Zahlenwert oder -bereich verwendet werden, +/-5% des angeführten Zahlenwerts oder -bereichs.

Im Rahmen der vorliegenden Offenbarung gelten als Millimeter-Radarwellen $f_S$ insbesondere elektromagnetische Wellen mit Wellenlängen zwischen zwei und achtunddreissig Millimetern, bevorzugt elektromagnetische Wellen mit Wellenlängen zwischen zwei und fünfundzwanzig Millimetern, speziell bevorzugt elektromagnetische Wellen mit Wellenlängen zwischen drei und siebzehn Millimetern.

Das Genannte bezieht sich auf einzelne Ausführungsformen der Offenbarung. Verschiedene Änderungen an den Ausführungsformen können vorgenommen werden, ohne von der zu Grunde liegenden Idee abzuweichen und ohne den Rahmen dieser Offenbarung zu verlassen. Der Gegenstand der vorliegenden Offenbarung ist definiert über deren Ansprüche. Es können verschiedenste Änderungen vorgenommen werden, ohne den Schutzbereich der folgenden Ansprüche zu verlassen.

**Patentansprüche**

1. Einrichtung zur Bestimmung des Mischungsverhältnisses eines Gemisches (FL), wobei das Gemisch (FL) zumindest zwei unterschiedliche Fluide (H20, GLY) umfasst, die Einrichtung umfassend:

   - einen Leitungsabschnitt (2) mit einem für die Bestimmung des Mischungsverhältnisses vorgesehenen, insbesondere durchströmten, Messbereich (MR) ;
   - wobei das Gemisch (FL) zum Durchströmen des Leitungsabschnittes (2) vorgesehen ist;
   - ein Radarsensorsystem (RS) umfassend einen Radarsensorchip (RC), wobei der Radarsensorchip (RC) eine Sensoraussenseite aufweist, welche an einer Aussenwandung des Leitungsabschnittes (2) angeordnet ist und/oder diese Aussenwandung durchdringt;

   wobei das Radarsensorsystem (RS) eingerichtet ist:

   - in einem vorgegebenen Frequenzbereich ($\Delta f$) frequenzmodulierte Millimeter-Radarwellen ($f_S$) über die Sensoraussenseite in den Messbereich (MR) einzustrahlen;
   - an dem Gemisch (FL) zurückgestreute Millimeter-Radarwellen ($f_R$) zu empfangen;
   - anhand der zurückgestreuten Millimeter-Radarwellen ($f_R$) einen frequenzabhängigen Reflexionsfaktor ($\rho_f$) für den vorgegebenen Frequenzbereich ($\Delta f$) zu ermitteln; und
   - das Mischungsverhältnis aus dem ermittelten frequenzabhängigen Reflexionsfaktor ($\rho_f$) rechnerisch zu bestimmen,
   **dadurch gekennzeichnet, dass**

   der Radarsensorchip (RC) an seiner Sensoraussenseite mindestens eine Empfangsantenne (Rx0 - Rx3) aufweist;
   wobei das Radarsensorsystem (RS) eingerichtet ist:

   - anhand der mindestens einen Empfangsantenne (Rx0 - Rx3) an dem Gemisch (FL) zurückgestreute Millimeter-Radarwellen ($f_R$) zu empfangen.

2. Die Einrichtung nach Anspruch 1, wobei der Radarsensorchip (RC) eingerichtet ist:

   - in einem vorgegebenen Frequenzbereich ($\Delta f$) frequenzmodulierte Millimeter-Radarwellen ($f_S$) über die Sensoraussenseite in den Messbereich (MR) einzustrahlen; und
   - an dem Gemisch (FL) zurückgestreute Millimeter-Radarwellen ($f_R$) zu empfangen.

3. Die Einrichtung nach einem der Ansprüche 1 bis 2,

   - wobei das Radarsensorsystem (RS) einen Mikrocontroller (MC) umfasst;
   - wobei der Mikrocontroller (MC) kommunikativ verbunden ist mit dem Radarsensorchip (RC);

   wobei der Mikrocontroller (MC) eingerichtet ist:

   - anhand der zurückgestreuten Millimeter-Radarwellen ($f_R$) einen frequenzabhängigen Reflexionsfaktor ($\rho_f$) für den vorgegebenen Frequenzbereich ($\Delta f$) zu ermitteln; und
   - das Mischungsverhältnis aus dem ermittelten frequenzabhängigen Reflexionsfaktor ($\rho_f$) rechnerisch zu bestimmen.

4. Die Einrichtung nach Anspruch 3, wobei der Mikrocontroller (MC) eingerichtet ist:

   - ein Detektionsergebnis (DET) umfassend Messwerte zu den zurückgestreuten Millimeter-Radarwellen ($f_R$) zu empfangen; und
   - anhand des Detektionsergebnisses (DET) einen frequenzabhängigen Reflexionsfaktor ($\rho_f$) für den vorgegebenen Frequenzbereich ($\Delta f$) zu

ermitteln.

**5.** Die Einrichtung nach einem der Ansprüche 3 bis 4,

- wobei das Radarsensorsystem (RS) einen Signalprozessor (SP) umfasst;
- wobei der Signalprozessor (SP) kommunikativ verbunden ist mit dem Radarsensorchip (RC);
- wobei der Signalprozessor (SP) kommunikativ verbunden ist mit dem Mikrocontroller (MC);

wobei der Signalprozessor (SP) eingerichtet ist:

- vom Radarsensorchip (RC) Empfangsdaten (RDAT) umfassend digitalisierte Signale zu den zurückgestreuten Millimeter-Radarwellen ($f_R$) zu empfangen;
- aus den Empfangsdaten (RDAT) ein Detektionsergebnis (DET), welches zu Messwerten verarbeitete digitalisierte Signale der Empfangsdaten (RDAT) umfasst, zu erzeugen;
- das Detektionsergebnis (DET) an den Mikrocontroller (MC) zu senden;

wobei der Mikrocontroller (MC) eingerichtet ist:

- das Detektionsergebnis (DET) vom Signalprozessor (SP) zu empfangen; und
- anhand des Detektionsergebnisses (DET) einen frequenzabhängigen Reflexionsfaktor ($\rho_f$) für den vorgegebenen Frequenzbereich ($\Delta f$) zu ermitteln.

**6.** Die Einrichtung nach Anspruch 5,
wobei der Mikrocontroller (MC) eingerichtet ist:

- Steuerdaten (CSP) an den Signalprozessor (SP) zu senden;
- wobei die Steuerdaten (CSP) mindestens eine Anweisung umfassen zur Einstrahlung frequenzmodulierter Millimeter-Radarwellen ($f_S$) im vorgegebenen Frequenzbereich ($\Delta f$);

wobei der Signalprozessor (SP) eingerichtet ist:

- zum Empfang der Steuerdaten (CSP) vom Mikrocontroller (MC);
- zur Erzeugung mindestens eines Steuersignales (CRC) aus den empfangenen Steuerdaten (CSP), wobei das mindestens eine Steuersignal (CRC) mindestens eine Grösse ausgewählt aus

  ∘ einer Frequenz,
  ∘ einem Frequenzhub,
  ∘ einem Modulationsverfahren umfasst;

- zum Senden des mindestens einen Steuersignales (CRC) an den Radarsensorchip (RC);

wobei der Radarsensorchip (RC) eingerichtet ist:

- zum Empfang des mindestens einen Steuersignales (CRC) vom Signalprozessor (SP);
- infolge des Empfanges des mindestens einen Steuersignales (CRC), zur Einstrahlung frequenzmodulierter Millimeter-Radarwellen ($f_S$) im vorgegebenen Frequenzbereich ($\Delta f$) über die Sensoraussenseite in den Messbereich (MR); und
- wobei die Einstrahlung als Funktion der mindestens einen von dem mindestens einen Steuersignal (CRC) umfassten Grösse erfolgt.

**7.** Die Einrichtung nach einem der Ansprüche 1 bis 6, wobei der Radarsensorchip (RC) an seiner Sensoraussenseite mindestens eine Sendeantenne (TxO, Tx1) aufweist;
wobei das Radarsensorsystem (RS) eingerichtet ist:

- in einem vorgegebenen Frequenzbereich ($\Delta f$) frequenzmodulierte Millimeter-Radarwellen (RADAR) über die Sensoraussenseite in den Messbereich (MR) anhand der mindestens einen Sendeantenne (TxO, Tx1) einzustrahlen.

**8.** Die Einrichtung nach einem der Ansprüche 1 bis 7, die Einrichtung zusätzlich umfassend:

- eine radarwellenabsorbierende Schicht (4); und
- wobei die radarwellenabsorbierende Schicht (4) an einer Aussenwandung des Leitungsabschnittes (2) angeordnet ist und/oder diese Aussenwandung durchdringt.

**9.** Die Einrichtung nach Anspruch 8,
wobei die radarwellenabsorbierende Schicht (4) eine Schicht aus radarwellenabsorbierendem Material (RAM) umfasst; und
wobei das radarwellenabsorbierendem Material (RAM) ein radarwellenabsorbierender Schaum ist.

**10.** Die Einrichtung nach Anspruch 8,
wobei die radarwellenabsorbierende Schicht (4) eine Schicht aus radarwellenabsorbierendem Material (RAM) umfasst; und
wobei die Schicht aus radarwellenabsorbierendem Material (RAM) Bällchen umfasst, welche mit Carbonyleisen beschichtet sind.

**11.** Die Einrichtung nach einem der Ansprüche 1 bis 10, wobei das Radarsensorsystem (RS) eingerichtet ist:

- in einem vorgegebenen Frequenzbereich (Df) frequenzmodulierte Millimeter-Radarwellen (fS) mit Wellenlängen zwischen drei und siebzehn Millimetern über die Sensoraussenseite in den Messbereich (MR) einzustrahlen.

**Claims**

1. Device for determining the mixing ratio of a mixture (FL), wherein the mixture (FL) comprises at least two different fluids (H20, GLY), the device comprising:

    - a pipe section (2) with a measuring region (MR), in particular one through which fluid flows, provided for determining the mixing ratio;
    - wherein the mixture (FL) is provided to flow through the pipe section (2);
    - a radar sensor system (RS) comprising a radar sensor chip (RC), wherein the radar sensor chip (RC) has a sensor outer side, which is arranged on an outer wall of the pipe section (2) and/or penetrates this outer wall;

    wherein the radar sensor system (RS) is adapted to:

    - irradiate frequency-modulated millimetre-radar waves ($f_S$) in a specified frequency range ($\Delta f$) via the sensor outer side into the measuring region (MR);
    - receive millimetre-radar waves ($f_R$) backscattered back at the mixture (FL);
    - ascertain a frequency-dependent reflection coefficient ($\rho_f$) for the specified frequency range ($\Delta f$) using the backscattered millimetre-radar waves ($f_R$); and
    - calculate the mixing ratio from the ascertained frequency-dependent reflection coefficient ($\rho_f$), **characterised in that**

    the radar sensor chip (RC) has at its sensor outer side at least one receiving antenna (Rx0 - Rx3); wherein the radar sensor system (RS) is adapted to:

    - receive millimetre-radar waves ($f_R$) backscattered at the mixture (FL) using the at least one receiving antenna (Rx0 - Rx3).

2. Device according to claim 1, wherein the radar sensor chip (RC) is adapted to:

    - irradiate frequency-modulated millimetre-radar waves ($f_S$) in a specified frequency range ($\Delta f$) via the sensor outer side into the measuring region (MR); and
    - receive millimetre-radar waves ($f_R$) backscattered at the mixture (FL).

3. Device according to one of claims 1 to 2,

    - wherein the radar sensor system (RS) comprises a microcontroller (MC);

    - wherein the microcontroller (MC) is communicatively connected to the radar sensor chip (RC);

    wherein the microcontroller (MC) is adapted to:

    - ascertain a frequency-dependent reflection coefficient ($\rho_f$) for the specified frequency range ($\Delta f$) using the backscattered millimetre-radar waves ($f_R$); and
    - calculate the mixing ratio from the ascertained frequency-dependent reflection coefficient ($\rho_f$).

4. Device according to claim 3, wherein the microcontroller (MC) is adapted to:

    - receive a detection result (DET) comprising measured values relating to the backscattered millimetre-radar waves ($f_R$); and
    - ascertain a frequency-dependent reflection coefficient ($\rho_f$) for the specified frequency range ($\Delta f$) using the detection result (DET).

5. Device according to one of claims 3 to 4,

    - wherein the radar sensor system (RS) comprises a signal processor (SP);
    - wherein the signal processor (SP) is communicatively connected to the radar sensor chip (RC);
    - wherein the signal processor (SP) is communicatively connected to the microcontroller (MC);

    wherein the signal processor (SP) is adapted to:

    - receive from the radar sensor chip (RC) received data (RDAT) comprising digitised signals relating to the backscattered millimetre-radar waves ($f_R$);
    - generate from the received data (RDAT) a detection result (DET), which comprises digitised signals of the received data (RDAT) processed to form measured values;
    - send the detection result (DET) to the microcontroller (MC) ;

    wherein the microcontroller (MC) is adapted to:

    - receive the detection result (DET) from the signal processor (SP); and
    - to ascertain a frequency-dependent reflection coefficient ($\rho_f$) for the specified frequency range ($\Delta f$) using the detection result (DET).

6. Device according to claim 5, wherein the microcontroller (MC) is adapted to:

- send control data (CSP) to the signal processor (SP);
- wherein the control data (CSP) comprises at least one instruction for the irradiation of frequency-modulated millimetre-radar waves ($f_S$) in the specified frequency range ($\Delta f$);

wherein the signal processor (SP) is adapted to:

- receive the control data (CSP) from the microcontroller (MC);
- generate at least one control signal (CRC) from the received control data (CSP), wherein the at least one control signal (CRC) comprises at least one variable selected from

  ∘ a frequency,
  ∘ a frequency deviation,
  ∘ a modulation method;

- send the at least one control signal (CRC) to the radar sensor chip (RC);

wherein the radar sensor chip (RC) is adapted to:

- receive the at least one control signal (CRC) from the signal processor (SP);
- as a result of receiving the at least one control signal (CRC), irradiate frequency-modulated millimetre-radar waves ($f_S$) in the specified frequency range ($\Delta f$) via the sensor outer side into the measuring region (MR); and
- wherein irradiation occurs as a function of the at least one variable incorporated by the at least one control signal (CRC).

7. Device according to one of claims 1 to 6, wherein the radar sensor chip (RC) has at its sensor outer side at least one transmitting antenna (TxO, Tx1); wherein the radar sensor system (RS) is adapted to:

- irradiate frequency-modulated millimetre-radar waves (RADAR) in a specified frequency range ($\Delta f$) via the sensor outer side into the measuring region (MR) using the at least one transmitting antenna (TxO, Tx1).

8. Device according to one of claims 1 to 7, the device also comprising:

- a radar wave-absorbing layer (4); and
- wherein the radar wave-absorbing layer (4) is arranged on an outer wall of the pipe section (2) and/or penetrates this outer wall.

9. Device according to claim 8, wherein the radar wave-absorbing layer (4) comprises a layer of radar wave-absorbing material (RAM); and wherein the radar wave-absorbing material (RAM) is a radar wave-absorbing foam.

10. Device according to claim 8, wherein the radar wave-absorbing layer (4) comprises a layer of radar wave-absorbing material (RAM); and wherein the layer of radar wave-absorbing material (RAM) comprises small balls, which are coated with carbonyl iron.

11. Device according to one of claims 1 to 10, wherein the radar sensor system (RS) is adapted to:

- irradiate frequency-modulated millimetre-radar waves (fS) with wavelengths between three and seventeen millimetres in a specified frequency range (Df) via the sensor outer side into the measuring region (MR).

**Revendications**

1. Dispositif pour la détermination du rapport de mélange d'un mélange (FL), dans lequel le mélange (FL) comprend au moins deux fluides différents (H2O, GLY), ce dispositif comprenant :

- une portion de conduite (2) avec une zone de mesure (MR) prévue, plus particulièrement traversée pour la détermination du rapport de mélange ;
- dans lequel le mélange (FL) est prévu pour traverser la portion de conduite (2) ;
- un système de détecteur radar (RS) comprenant une puce de détecteur radar (RC), dans lequel la puce de détecteur radar (RC) présente un côté externe de détecteur, qui est disposée sur une paroi externe de la portion de conduite (2) et/ou traverse cette paroi externe ;

dans lequel le système de détecteur radar (RS) est conçu :

- pour émettre, dans une plage de fréquence ($\Delta f$) prédéterminée, des ondes radar millimétriques modulées en fréquence ($f_S$) par l'intermédiaire du côté externe de détecteur, dans la zone de mesure (MR) ;
- pour recevoir, des ondes radar millimétriques ($f_R$) réfléchies au niveau du mélange (FL) ;
- pour déterminer, à l'aide des ondes radar millimétriques ($f_R$) réfléchies, un facteur de réflexion dépendant de la fréquence ($\rho_f$) pour la plage de fréquence ($\Delta f$) prédéterminée ; et
- pour déterminer par calcul le rapport de mélange à partir du facteur de réflexion dépendant de la fréquence ($\rho_f$) déterminé,

**caractérisé en ce que**

la puce de détecteur radar (RC) comprend, sur son côté externe de détecteur, au moins une antenne de réception (Rx0 - Rx3) ;

dans lequel le système de détecteur radar (RS) est conçu :

- pour recevoir, à l'aide de l'au moins une antenne de réception (Rx0 - Rx3), les ondes radar millimétriques ($f_R$) réfléchies au niveau du mélange (FL).

2. Dispositif selon la revendication 1, dans lequel la puce de détecteur radar (RC) est conçue :

- pour émettre, dans une plage de fréquence ($\Delta f$) prédéterminée, des ondes radar millimétriques modulées en fréquence ($f_S$) par l'intermédiaire du côté externe de détecteur, dans la zone de mesure (MR) ; et
- pour recevoir des ondes radar millimétriques ($f_R$) réfléchies au niveau du mélange (FL).

3. Dispositif selon l'une des revendications 1 à 2,

- dans lequel le système de détecteur radar (RS) comprend un microcontrôleur (MC) ;
- dans lequel le microcontrôleur (MC) est relié en communication avec la puce de détecteur radar (RC) ;

dans lequel le microcontrôleur (MC) est conçu :

- pour déterminer, à l'aide des ondes radar millimétriques ($f_R$) réfléchies, un facteur de réflexion dépendant de la fréquence ($\rho_f$) pour la plage de fréquence ($\Delta f$) prédéterminée ; et
- pour déterminer par calcul le rapport de mélange à partir du facteur de réflexion dépendant de la fréquence ($\rho_f$) déterminé.

4. Dispositif selon la revendication 3, dans lequel le microcontrôleur (MC) est conçu :

- pour recevoir un résultat de détection (DET) comprenant des valeurs de mesure concernant les ondes radar millimétriques ($f_R$) réfléchies ; et
- pour déterminer, à l'aide du résultat de détection (DET), un facteur de réflexion dépendant de la fréquence ($\rho_f$) pour la plage de fréquence ($\Delta f$) prédéterminée.

5. Dispositif selon l'une des revendications 3 à 4,

- dans lequel le système de détecteur radar (RS) comprend un processeur de signaux (SP) ;
- dans lequel le processeur de signaux (SP) est relié en communication avec la puce de détecteur radar (RC) ;
- dans lequel le processeur de signaux (SP) est relié en communication avec le microcontrôleur (MC) ;

dans lequel le processeur de signaux (SP) est conçu :

- pour recevoir, en provenance de la puce de détecteur radar (RC), des données de réception (RDAT) comprenant des signaux numérisés concernant les ondes radar millimétriques ($f_R$) réfléchies ;
- pour générer, à partir des données de réception (RDAT), un résultat de détection (DET), qui comprend des signaux numérisés des données de réception (RDAT), traités afin d'obtenir des valeurs de mesure ;
- pour envoyer le résultat de détection (DET) au microcontrôleur (MC) ;

dans lequel le microcontrôleur (MC) est conçu :

- pour recevoir le résultat de détection (DET) en provenance du processeur de signaux (SP) ; et
- pour déterminer, à l'aide du résultat de détection (DET), un facteur de réflexion dépendant de la fréquence ($\rho_f$) pour la plage de fréquence ($\Delta f$) prédéterminée.

6. Dispositif selon la revendication 5, dans lequel le microcontrôleur (MC) est conçu :

- pour envoyer des données de contrôle (CSP) au processeur de signaux (SP) ;
- dans lequel les données de contrôle (CSP) comprennent une instruction pour l'émission d'ondes radar millimétriques modulées en fréquence ($f_S$) dans la plage de fréquence ($\Delta f$) prédéterminée ;

dans lequel le processeur de signaux (SP) est conçu :

- pour la réception des données de contrôle (CSP) en provenance du microcontrôleur (MC) ;
- pour la production d'au moins un signal de contrôle (CRC) à partir des données de contrôle (CSP) reçues, dans lequel l'au moins un signal de contrôle (CRC) présente une grandeur sélectionnée parmi

• une fréquence,
• un balayage de fréquences,
• un procédé de modulation ;

- pour l'envoi de l'au moins un signal de contrôle (CRC) à la puce de détecteur radar (RC) ;

dans lequel la puce de détecteur radar (RC) est conçue :

- pour la réception de l'au moins un signal de contrôle (CRC) en provenance du processeur de signaux (SP) ;
- à la suite de la réception de l'au moins un signal de contrôle (CRC), pour l'émission d'ondes radar millimétriques modulées en fréquence ($f_S$) dans la plage de fréquence ($\Delta f$) prédéterminée, par l'intermédiaire du côté externe du détecteur, dans la zone de mesure (MR) ; et
- dans lequel l'émission a lieu en fonction de l'au moins une grandeur présentée par l'au moins un signal de contrôle (CRC).

7. Dispositif selon l'une des revendications 1 à 6, dans lequel la puce de détecteur radar (RC) comprend, sur son côté externe de détecteur, au moins une antenne d'émission (TxO, Tx1) ; dans lequel le système de détecteur radar (RS) est conçu :

- pour émettre, dans une plage de fréquence ($\Delta f$) prédéterminée, des ondes radar millimétriques modulées en fréquence (RADAR), par l'intermédiaire du côté externe du détecteur, dans la zone de mesure (MR), à l'aide de l'au moins une antenne d'émission (TxO, Tx1).

8. Dispositif selon l'une des revendications 1 à 7, ce dispositif comprenant en outre :

- une couche absorbant les ondes radar (4) ; et
- dans lequel la couche absorbant les ondes radar (4) est disposée sur une paroi externe de la portion de conduite (2) et/ou traverse cette paroi externe.

9. Dispositif selon la revendication 8, dans lequel la couche absorbant les ondes radar (4) comprend une couche en matériau absorbant les ondes radar (RAM) ; et dans lequel le matériau absorbant les ondes radar (RAM) est une mousse absorbant les ondes radar.

10. Dispositif selon la revendication 8, dans lequel la couche absorbant les ondes radar (4) comprend une couche en matériau absorbant les ondes radar (RAM) ; et dans la couche de matériau absorbant les ondes radar (RAM) comprend des billes qui sont revêtues de fer carbonyle.

11. Dispositif selon l'une des revendications 1 à 10,

dans lequel le système de détecteur radar (RS) est conçu :

- pour émettre, dans une plage de fréquence (Df) prédéterminée, des ondes radar millimétriques modulées en fréquence ($f_S$), avec des longueurs d'ondes entre trois et dix-sept millimètres, par l'intermédiaire du côté externe de détecteur, dans la zone de mesure (MR).

# FIG 1

EP 3 783 343 B1

# FIG 2

FIG 3

EP 3 783 343 B1

FIG 4

EP 3 783 343 B1

FIG 5

EP 3 783 343 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012065276 A1
- DE 102007015609 A1
- WO 2018078401 A1
- US 2009126502 A1
- EP 2520148 A2

**In der Beschreibung aufgeführte Nicht-Patentliteratur**

- **JAIME LIEN ; NICHOLAS GILLIAN ; M. EMRE KARAGOZLER ; PATRICK AMIHOOD ; CARSTEN SCHWESIG ; ERIK OLSON ; HAKIM RAJA ; IVAN POUPYREV.** Soli: Ubiquitous Gesture Sensing with Millimeter Wave Radar. *ACM Transactions on Graphics,* Juli 2016, vol. 35 (4